# FASCICULE DE BREVET EUROPEEN

(11) **EP 2 175 985 B1**
(45) Date de publication et mention de la délivrance du brevet: **08.05.2019**
(21) Numéro de dépôt: 08826442.9
(22) Date de dépôt: 18.06.2008
(51) Int. Cl.: B01J 20/18, B01J 20/30, C10G 25/05, B01D 53/04

(54) **PURIFICATION D'UNE COUPE OLÉFINIQUE PAR ADSORPTION SUR DES COGRANULES ALUMINE-FAUJASITE**
REINIGUNG EINES OLEFINISCHEN SCHNITTS DURCH ADSORPTION EINES ALUMINIUMOXID-FAUJASITH COGRANULATS
PURIFICATION OF AN OLEFIN CUT BY ADSORPTION ON ALUMINA/FAUJASITE COMPOSITE GRANULES

(30) Priorité: 06.07.2007 FR 0704940
(43) Date de publication de la demande: 21.04.2010
(73) Titulaire: IFP Energies nouvelles, 92500 Rueil-Malmaison (FR)
(72) Inventeur: DUCREUX, Olivier, F-78380 Bougival (FR); NEDEZ, Christophe, F-30340 Salindres (FR); POMMIER, Catherine, F-30340 Saint-Julien-Les Rosiers (FR)
(74) Mandataire: IFP Energies nouvelles
(86) Numéro de dépôt international: PCT/FR2008/000852
(87) Numéro de publication internationale: WO 2009/010666

(56) Documents cités:
- FR-A- 2 817 772
- US-A- 3 899 310
- US-B1- 6 638 340

## Description

**La présente invention concerne** un procédé d'adsorption de contaminants organiques comprenant au moins un hétéroatome sélectionné dans le groupe constitué par l'azote, le soufre et l'oxygène, ces contaminants organiques étant présents dans une charge oléfinique qui comprend au moins 50% volume d'hydrocarbures, ledit procédé d'adsorption comprenant l'étape de mise en contact de ladite charge oléfinique avec un adsorbant obtenu par la méthode de préparation comprenant les étapes successives suivantes : A) une étape A de mise en forme par cogranulation d'une poudre A de zéolithe de type faujasite, avec une poudre B composée d'alumine, la proportion massique de la poudre A dans le mélange des poudres A et B étant comprise entre 10 et 70%, B) une étape B de traitement sous vapeur d'eau de l'adsorbant obtenu à l'issue de l'étape A à une température comprise entre 60 et 120°C, pendant une durée de 1 à 12 heures, C) une étape C de séchage de l'adsorbant obtenue à l'issue de l'étape B à une température comprise entre 100 et 300°C pendant une durée de 0,5 à 12 heures.

### ART ANTÉRIEUR

L'art antérieur décrit des méthodes de préparation de cogranulés faujasite-alumine.

Il existe déjà des procédés de préparation avec un agglomérant de zéolithes de type A et de type faujasite qui sont basés sur la préparation de la masse réactionnelle par mélange d'une solution aqueuse de silicate de sodium avec une solution aqueuse d'aluminate de sodium et formation dans ce cas d'un sol qui coagule spontanément en gel, ce dernier étant ensuite soumis à la cristallisation. Il se forme dans ce cas une zéolithe cristalline finement dispersée qui, après cristallisation, doit être séparée de la liqueur mère, lavée à l'eau et soumise au formage avec addition d'un agglomérant en granulés et enfin séchée et calcinée. (voir par exemple les brevets US2882243 et US3234147).

Il existe aussi des procédés de préparation sans agglomérants de zéolithes synthétiques sous forme de granulés. De tels procédés consistent à préparer des masses réactionnelles telles que des pâtes alcalines d'aluminosilicates que l'on transforme en granulés. On les soumet ensuite à la cristallisation. Après cristallisation, on lave les granulés à l'eau et on les sèche (voir par exemple le brevet US3094383).

### DESCRIPTION DÉTAILLÉE DE L'INVENTION

La méthode de préparation d'un adsorbant comprent les étapes successives suivantes:
A) une étape A de mise en forme par cogranulation d'une poudre A de zéolithe de type faujasite, avec une poudre B composée d'alumine, la proportion massique de la poudre A dans le mélange des poudres A et B étant comprise entre 10 et 70%, préférentiellement comprise entre 15 et 60%, de manière très préférée comprise entre 20 et 60%, de manière plus préférée comprise entre 21 et 57%, de manière encore plus préférée comprise entre 35 et 57%, de manière encore plus préférée comprise entre 40 et 57%, voire de manière encore plus préférée comprise entre 45 et 55%.
B) une étape B de traitement sous vapeur d'eau de l'adsorbant obtenu à l'issue de l'étape A à une température comprise entre 60 et 120°C, pendant une durée de 1 à 12 heures, de façon très préférée de 2 à 11 heures, de manière très préférée de 3 à 7 heures.
C) une étape C de séchage de l'adsorbant obtenu à l'issue de l'étape B à une température comprise entre 100 et 300°C, de préférence entre 150 et 250°C, de manière très préférée entre 190 et 210°C, pendant une durée de 0,5 à 12 heures, de préférence de 1 à 10 heures, de façon très préférée de 1 à 4 heures, de manière très préférée de 1 à 3 heures, de manière plus préférée de 1,5 à 2,5 heures.

Dans le cadre de l'invention, on parlera d'adsorbant ou de co-granulés pour désigner le même solide.

Un mélange des deux poudres A et B est généralement pratiqué avant mise en forme de l'ensemble. Il est réalisé préférentiellement au moyen d'une technologie tournante connue par l'homme du métier comme, par exemple, la granulation dans un drageoir ou dans un tambour.

Optionnellement, l'étape C de séchage est suivie d'une étape D de calcination de l'adsorbant à une température située entre 200 et 700°C, de préférence entre 250 et 600°C, encore préférentiellement entre 250 et 500°C. La durée de l'étape D de calcination est généralement comprise entre 1 et 7 heures, de préférence entre 1 et 5 heures.

Généralement, la zéolithe est de type faujasite est une zéolithe NaX.

Selon une variante préférée, la poudre B est obtenue par le procédé connu par l'homme du métier de déshydratation rapide d'un hydroxyde d'alumine, de préférence l'hydrargillite.

Généralement, l'adsorbant est dopé par un ou plusieurs éléments sélectionnés dans le groupes constitués par:
- les oxydes d'alcalins
- les oxydes d'alcalino-terreux
- les oxydes de terres rares.

La teneur massique de l'adsorbant en oxydes d'éléments dopants correspond par définition à la teneur massique totale en oxydes d'éléments dopants à laquelle est retranchée la teneur massique en oxyde de sodium apporté par la poudre A et elle est généralement inférieure à 20%, de préférence inférieure à 10%, avantageusement comprise entre 500 ppm et 5%.

Les dopants sont généralement ajoutés par imprégnation à sec de la poudre A ou de la poudre B, ou de la poudre A et de la poudre B avant l'étape A de mise en forme ou par imprégnation à sec de l'adsorbant au cours ou à l'issue de l'étape A.

Selon une méthode préférée, les dopants sont ajoutés à au moins une des deux poudres utilisées, avant de procéder à l'étape A de mise en forme.

La surface spécifique dudit adsorbant est généralement comprise entre 300 et 900 m²/g, de préférence comprise entre 300 et 600 m²/g, de préférence entre 320 et 580 m²/g voire comprise entre 350 et 550 m²/g. L'adsorbant se présente généralement sous toutes les formes habituelles connues par l'homme de l'art telles que par exemple de la poudre, des billes, des extrudés, des concassés, des monolithes. De manière préférée, l'adsorbant est mis en forme sous forme de billes lors de l'étape A. La taille des billes est alors généralement comprise bornes incluses entre 0,5 et 10 mm, de préférence comprise bornes incluses entre 0,7 et 8 mm, encore préférentiellement comprise bornes incluses entre 0,8 et 5 mm.

L'invention concerne un procédé d'adsorption de contaminants organiques contenant au moins un hétéroatome et présents dans une charge oléfinique comprenant au moins 50% volume d'hydrocarbures, de préférence 60% volume d'hydrocarbures, voire 70% volume d'hydrocarbures, voire 80% volume d'hydrocarbures. Ce procédé comprend l'étape de mise en contact de ladite charge oléfinique avec l'adsorbant obtenu par une des méthodes de préparation selon l'invention, cette mise en contact étant réalisée de préférence en conditions dynamiques, l'adsorbant étant généralement disposé en lit fixe. La charge oléfinique comprend généralement des d'hydrocarbures comprenant de 2 à 12 atomes de carbone et plus particulièrement de 3 à 10 atomes de carbone et plus spécialement de 3 à 7 atomes de carbone. La charge oléfinique provient généralement d'un vapocraqueur ou d'un craquage catalytique fluidisé et la teneur volumique globale en oléfines dans la charge est généralement comprise entre 20 et 100%, de préférence entre 40 et 80% d'oléfines et de manière plus préférée entre 40 et 60%.

Les contaminants organiques comprennent généralement au moins un composé comprenant au moins un hétéroatome sélectionné dans le groupe constitué par l'azote, le soufre et l'oxygène. Les contaminants organiques comprennent généralement au moins un composé sélectionné dans le groupe constitué par les nitriles, le pyrrole, le diméthylformamide, l'aniline, le méthylmercaptan, l'éthylmercaptan, le propylmercaptan, les soufrés, le thiophène, les cétones, les aldéhydes, les éthers et les alcools, de préférence au moins un composé sélectionné dans le groupe constitué par l'acétonitrile, le diméthylformamide, l'acétone, l'aniline, le méthylmercaptan et le méthanol.

Généralement, les nitriles correspondent à l'acétonitrile et au propionitrile. Généralement, les mercaptans légers correspondent au méthylmercaptan, à l'éthylmercaptan, voire au propylmercaptan. Généralement, les composés soufrés correspondent à l'H₂S, au COS, au CS₂, au diméthyldisulfure DMDS ou au diéthyldisulfure DEDS. Généralement, les cétones correspondent à l'acétone. Généralement, les aldéhydes correspondent à l'acétaldéhyde. Généralement, les éthers correspondent au MTBE, à l'ETBE. Généralement, les alcools correspondent au méthanol ou à l'éthanol. Les contaminants organiques comprennent généralement l'une quelconque de ces molécules, ou un mélange de plusieurs de ces molécules.

Généralement les co-granulés préparés peuvent être mis en oeuvre dans des procédés industriels continus ou non continus (procédés batchs par exemple), optionnellement avec une étape de régénération. Le procédé d'adsorption modulé un température TSA (Temperature Swing Asdorption selon la terminologie anglo-saxonne) est généralement employé.

### EXEMPLES

### Exemple 1

**Tableau I. Caractéristiques des adsorbants étudiés**

| Caractéristiques | Adsorbants | | | | | |
|---|---|---|---|---|---|---|
| | C | D | E | F | G | H |
| Forme | Billes | Billes | Billes | Billes | Billes | Billes |
| A (%poids) | 0 | 5 | 30 | 50 | 85 | 30 |
| B (%poids) | 100 | 95 | 70 | 50 | 15 | 70 |
| Diamètre (mm) | 2-5 | 2-5 | 2-5 | 2-5 | 2-5 | 2-5 |
| Surface spécifique (m²/g) | 342 | 359 | 424 | 454 | 609 | 339 |
| Volume poreux total (ml/100g) | 44,3 | 45,7 | 51,3 | 62,5 | 71,4 | 47,0 |
| teneur massique en Na₂O (%poids) | 0,35 | 0,33 | 0,25 | 0,18 | 0,05 | 2,15 |

La teneur massique de l'adsorbant en oxydes d'éléments dopants (ici teneur massique en Na₂O) correspond à la teneur massique total en oxydes d'éléments dopants à laquelle est retranchée la teneur massique en oxyde de sodium apporté par la poudre A.

Les solides D à G sont préparés par cogranulation, en drageoir, d'une poudre de zéolithe NaX (poudre A) et d'une poudre B, résultant de la déshydratation d'hydrargillite, en diverses proportions indiquées dans le Tableau I.

Après mise en forme, un traitement sous vapeur d'eau est assuré à 100°C durant 6 heures, avant séchage assuré à 200°C pendant 2 heures et calcination à 400°C durant 2 heures.

Le solide C est obtenu par granulation simple de la poudre B ; les mêmes traitements thermiques postérieurs que ceux décrits dans les cas D à G sont suivis.

Le solide H résulte d'une imprégnation, dite à sec, de soude effectuée sur les billes E préalablement obtenues. Un séchage est ensuite mené durant 2 heures à 200°C suivi d'une calcination à 400°C pendant 2 heures.

### Exemple 2

Des expériences d'adsorption ont été conduites en milieu liquide. L'étude a été assurée sur 1 gramme d'adsorbant, préalablement régénéré sous flux d'azote à 300°C durant 2 heures, puis placé dans un erlenmeyer en verre contenant 300 ml de 1-dodécène avec une concentration déterminée d'une impureté choisie.

L'erlenmeyer est fermé hermétiquement par un bouchon en verre rodé afin d'éviter toute évaporation parasite et il est maintenu à la température désirée au moyen d'un bain marie.

La diminution de la concentration de la solution organique en l'impureté est suivie par UV-visible ou par chromatographie en phase gazeuse et permet de déterminer la reprise de masse de l'adsorbant en l'impureté considérée.

Les résultats obtenus sont indiqués dans les Tableaux II et III. Ils illustrent l'intérêt de mettre en oeuvre les adsorbants E, F et G selon l'invention par rapport aux adsorbants C, D et G selon l'art antérieur. En effet, les adsorbants selon l'invention captent mieux les contaminants organiques

**Tableau II. Reprise de masse (en %) observée pour 1 gramme d'adsorbant exposé à une solution de 300 ml de 1-dodécène admettant 400 ppm d'aniline, en fonction du temps de réaction et de la température du milieu**

| Conditions | Adsorbants | | | | | |
|---|---|---|---|---|---|---|
| | C | D | E | F | G | H |
| 400 ppm d'aniline, 30 h à 25°C | 4,4 | 4,4 | 8,7 | 11,1 | 3,9 | 9,8 |
| 400 ppm d'aniline, 30 h à 35°C | 3,8 | 3,9 | 7,4 | 9,8 | 3,0 | 8,5 |
| 400 ppm d'aniline, 3 h à 45°C | 2,4 | 2,5 | 4,9 | 6,1 | 1,8 | 5,5 |
| 400 ppm d'aniline, 30 h à 45°C | 3,6 | 3,8 | 5,6 | 6,9 | 2,5 | 6,4 |

**Tableau III. Reprise de masse (en %) observée pour 1 gramme d'adsorbant exposé à une solution de 300 ml de 1-dodécène admettant 200 ppm de diméthylformamide (DMF) ou 200 ppm d'acétonitrile, après 30 heures de réaction à 25°C**

| Conditions | Adsorbants | | | | | |
|---|---|---|---|---|---|---|
| | C | D | E | F | G | H |
| 200 ppm de DMF, 30 h à 25°C | 2,9 | 3,2 | 5,4 | 6,8 | 3,5 | 5,8 |
| 200 ppm d'acétonitrile, 30 h à 25°C | 1,9 | 1,7 | 3,7 | 4,8 | 2,4 | 4,3 |

## Revendications

1. Procédé d'adsorption de contaminants organiques comprenant au moins un hétéroatome sélectionné dans le groupe constitué par l'azote, le soufre et l'oxygène, ces contaminants organiques étant présents dans une charge oléfinique qui comprend au moins 50% volume d'hydrocarbures, ledit procédé d'adsorption comprenant l'étape de mise en contact de ladite charge oléfinique avec un adsorbant obtenu par la méthode de préparation comprenant les étapes successives suivantes :
A) une étape A de mise en forme par cogranulation d'une poudre A de zéolithe de type faujasite, avec une poudre B composée d'alumine, la proportion massique de la poudre A dans le mélange des poudres A et B étant comprise entre 10 et 70%,
B) une étape B de traitement sous vapeur d'eau de l'adsorbant obtenu à l'issue de l'étape A à une température comprise entre 60 et 120°C, pendant une durée de 1 à 12 heures,
C) une étape C de séchage de l'adsorbant obtenue à l'issue de l'étape B à une température comprise entre 100 et 300°C pendant une durée de 0,5 à 12 heures.

2. Procédé d'adsorption selon la revendication 1 dans lequel ladite charge oléfinique comprend des d'hydrocarbures comprenant de 2 à 12 atomes de carbone.

3. Procédé d'adsorption selon l'une des revendications 1 ou 2 dans lequel ladite charge oléfinique provient d'un vapocraqueur ou d'un craquage catalytique fluidisé et dans lequel la teneur volumique globale en oléfines dans ladite charge est comprise entre 20 et 100%.

4. Procédé d'adsorption selon l'une des revendications 1 à 3 dans lequel lesdits contaminants organiques comprennent au moins un composé sélectionné dans le groupe constitué par les nitriles, le pyrrole, le diméthylformamide, l'aniline, le méthylmercaptan, l'éthylmercaptan, le propylmercaptan, les soufrés, le thiophène, les cétones, les aldéhydes, les éthers et les alcools.

5. Procédé d'adsorption selon l'une quelconque des revendications 1 à 4 dans laquelle l'étape C de séchage est suivie d'une étape D de calcination de l'adsorbant à une température située entre 200 et 700°C.

6. Procédé d'adsorption selon l'une des revendications 1 à 5 dans laquelle la zéolithe de type faujasite est une zéolithe NaX.

7. Procédé d'adsorption selon l'une des revendications 1 à 6 dans laquelle l'adsorbant est dopé par un ou plusieurs éléments sélectionnés dans le groupes constitués par les oxydes d'alcalins, les oxydes d'alcalino-terreux et les oxydes de terres rares.

8. Procédé d'adsorption selon la revendication 7 dans laquelle la teneur massique de l'adsorbant en oxydes d'éléments dopants est inférieure à 20%.

9. Procédé d'adsorption selon l'une des revendications 7 ou 8 dans laquelle les dopants sont ajoutés par imprégnation à sec de la poudre A ou de la poudre B, ou de la poudre A et de la poudre B avant l'étape A de mise en forme ou par imprégnation à sec de l'adsorbant au cours de l'étape A ou à la suite de l'étape A.

10. Procédé d'adsorption selon l'une des revendications précédentes dans laquelle l'adsorbant est mis en forme sous forme de billes lors de l'étape A.

## Patentansprüche

1. Verfahren zur Adsorption von organischen Kontaminanten, umfassend mindestens ein Heteroatom, ausgewählt aus der Gruppe bestehend aus Stickstoff, Schwefel und Sauerstoff, wobei diese organischen Kontaminanten in einem olefinischen Einsatzmaterial vorhanden sind, das mindestens 50 Vol.-% Kohlenwasserstoffe umfasst, wobei das Adsorptionsverfahren den Schritt zum Inkontaktbringen des olefinischen Einsatzmaterials mit einem Adsorptionsmittel umfasst, das durch das Herstellungsverfahren erhalten wird, das nacheinander die folgenden Schritte umfasst:
A) einen Schritt A zum Formen durch Co-Granulieren eines Pulvers A aus einem Zeolithen vom Typ Faujasit mit einem Pulver B, das aus Aluminiumoxid besteht, wobei der Gewichtsanteil des Pulvers A in der Mischung aus Pulver A und Pulver B im Bereich zwischen 10 und 70 % liegt,
B) einen Schritt B zum Behandeln unter Wasserdampf des Adsorptionsmittels, das am Ende von Schritt A erhalten wurde, bei einer Temperatur im Bereich zwischen 60 und 120 °C für eine Dauer von 1 bis 12 Stunden,
C) einen Schritt C zum Trocknen des Adsorptionsmittels, das am Ende von Schritt B erhalten wurde, bei einer Temperatur im Bereich zwischen 100 und 300 °C für eine Dauer von 0,5 bis 12 Stunden.

2. Adsorptionsverfahren nach Anspruch 1, wobei das olefinische Einsatzmaterial Kohlenwasserstoffe mit 2 bis 12 Kohlenstoffatomen umfasst.

3. Adsorptionsverfahren nach einem der Ansprüche 1 oder 2, wobei das olefinische Einsatzmaterial aus einem Dampfcracker oder einem katalytischen Wirbelschichtkracken stammt und wobei der Gesamtgehalt an Olefinen in dem Einsatzmaterial im Bereich zwischen 20 und 100 Vol.-% liegt.

4. Adsorptionsverfahren nach einem der Ansprüche 1 bis 3, wobei die organischen Kontaminanten mindestens eine Verbindung umfassen, ausgewählt aus der Gruppe bestehend aus Nitrilen, Pyrrol, Dimethylformamid, Anilin, Methylmercaptan, Ethylmercaptan, Propylmercaptan, Schwefelverbindungen, Thiophenen, Ketonen, Aldehyden, Ethern und Alkoholen.

5. Adsorptionsverfahren nach irgendeinem der Ansprüche 1 bis 4, wobei auf den Schritt C zum Trocknen ein Schritt D zum Kalzinieren des Adsorptionsmittels bei einer Temperatur zwischen 200 und 700 °C folgt.

6. Adsorptionsverfahren nach einem der Ansprüche 1 bis 5, wobei der Zeolith von Typ Faujasit ein NaX-Zeolith ist.

7. Adsorptionsverfahren nach einem der Ansprüche 1 bis 6, wobei das Adsorptionsmittel mit einem oder mehreren Elementen dotiert ist, ausgewählt aus den Gruppen bestehend aus Alkalioxiden, Erdalkalioxiden und Seltenerdenoxiden.

8. Adsorptionsverfahren nach Anspruch 7, wobei der Gewichtsanteil der Oxide der Dotierelemente in dem Adsorptionsmittel weniger als 20 % beträgt.

9. Adsorptionsverfahren nach einem der Ansprüche 7 oder 8, wobei die Dotiermittel durch Trockenimprägnierung des Pulvers A oder des Pulvers B oder des Pulvers A und des Pulvers B vor dem Schritt A zum Formen oder durch Trockenimprägnierung des Adsorptionsmittels im Verlauf von Schritt A oder im Anschluss an Schritt A zugegeben werden.

10. Adsorptionsverfahren nach einem der vorhergehenden Ansprüche, wobei das Adsorptionsmittel im Verlauf von Schritt A in Form von Kugeln geformt wird.

## Claims

1. Process for adsorption, of organic contaminants that comprise at least one heteroatom that is selected from the group that consists of nitrogen, sulfur and oxygen, whereby these organic contaminants were present in an olefinic feedstock that comprises at least 50% by volume of hydrocarbons, whereby said adsorption process comprises the stage for putting said olefinic feedstock in contact with an adsorbent obtained according to the preparation method that comprises the following successive stages:
A) a stage A for shaping a faujasite-type zeolite powder A by co-granulation with a powder B that consists of alumina, whereby the ratio per unit of mass of the powder A in the mixture of powders A and B is between 10 and 70%,
B) a stage B for treatment of the adsorbent that is obtained at the end of stage A under water vapor at a temperature of between 60 and 120°C, for a duration of 1 to 12 hours,
C) a stage C for drying the adsorbent that is obtained at the end of stage B at a temperature of between 100 and 300°C for a duration of 0.5 to 12 hours.

2. Adsorption process according to claim 1, wherein said olefinic feedstock comprises hydrocarbons that comprise 2 to 12 carbon atoms.

3. Adsorption process according to one of claims 1 or 2, wherein said olefinic feedstock originates from a stream-cracking device or a fluidized catalytic cracking and in which the overall volumetric content of olefins in said feedstock is between 20 and 100%.

4. Adsorption process according to one of claims 1 to 3, wherein said organic contaminants comprise at least one compound that is selected from the group that consists of nitriles, pyrrole, dimethylformamide, aniline, methyl mercaptan, ethyl mercaptan, propyl mercaptan, sulfur compounds, thiophenes, ketones, aldehydes, ethers, and alcohols.

5. Adsorption process according to one of claims 1 to 4, wherein the drying stage C is followed by a stage D for calcination of the adsorbent at a temperature that is between 200 and 700°C.

6. Adsorption process according to one of claims 1 to 5, wherein the faujasite-type zeolite is an NaX zeolite.

7. Adsorption process according to one of claims 1 to 6, wherein the adsorbent is doped by one or more elements that are selected from the groups that consist of alkaline oxides, alkaline-earth oxides, and rare earth oxides.

8. Adsorption process according to claim 7, wherein the content by mass of the adsorbent in oxides of doping elements is less than 20%.

9. Adsorption process according to one of claims 7 or 8, wherein the dopants are added by dry impregnation of the powder A or of the powder B, or powder A and powder B before the shaping stage A or by dry impregnation of the adsorbent during stage A or following stage A.

10. Adsorption process according to one of the preceding claims, wherein the adsorbent is shaped in the form of balls during stage A.
